# EUROPEAN PATENT APPLICATION

(11) **EP 4 349 990 A1**
(43) Date of publication of application: **10.04.2024**
(21) Application number: 22382946.6
(22) Date of filing: 07.10.2022
(51) Int. Cl.: C12N 15/67

(54) **ARTIFICIAL POLYNUCLEOTIDES FOR EXPRESSING PROTEINS**

(71) Applicant: Certest Biotec, S.L., 50840 San Mateo De Gallego (Zaragoza) (ES)
(72) Inventor: BROSET BLASCO, Esther, 50840 SAN MATEO DE GÁLLEGO (ES); MARTÍNEZ OLIVÁN, Juan Enrique, 50840 SAN MATEO DE GÁLLEGO (ES)
(74) Representative: ZBM Patents - Zea, Barlocci & Markvardsen

(57) **Abstract**

The present invention provides a polynucleotide comprising, in the 5' to 3' direction, a 5' untranslated region (5'-UTR) and an open reading frame (ORF), wherein the 5'-UTR comprises at least two tandem repeats of sequence 5'-GCCNCC-3' operatively linked to the ORF, and wherein N is any nucleotide. The invention also provides a composition comprising a lipid nanoparticle and the polynucleotide and a pharmaceutical composition, and their use in medicine, particularly for use as a vaccine or for use in gene therapy.

## Description

### Technical Field

The present invention belongs to the field of polynucleotides, in particular artificial polynucleotides encoding polypeptides. The polynucleotides of the present invention are particularly useful for genetic vaccination.

### Background Art

Genetic vaccination and gene therapy represent two of the most promising and quickly developing therapeutic areas of modern medicine. They are both based on the delivery of polynucleotides -such as DNA or RNA molecules- into a patient's cells or tissue for producing a polypeptide that confers a therapeutic benefit.

The use of RNA in gene therapy and vaccination is generally considered safer than the use of DNA, since RNA does not involve the risk of being stably integrated into the genome of the transfected cell. In addition, RNA is much easier to degrade *in vivo,* resulting in a relatively short half-life in contrast with DNA. Thus, compared to DNA-based therapies, RNA-based therapies have a lower risk of generating undesired anti-RNA antibodies by the host that could reduce therapy efficacy and produce serious side effects. Therefore, RNA is considered in many cases as the molecule of choice for gene medical therapies.

One of the main limitations of RNA-based therapies is their limited protein production efficiency. Because RNAs have a relatively short half-life, it is crucial that they provide exceptionally high translation rates in order to produce sufficient amounts of the desired protein before the RNAs are degraded. Optimization of UTRs to improve protein production has been attempted by rational design of 5'UTRs; however, the advances achieved so far are still very limited.

Thus, despite the efforts made in recent years, there is still a need for polynucleotides, in particular RNAs, with high translation rates suitable for use in genetic vaccination and gene therapy.

### Summary of Invention

The present inventors have developed a new nucleic acid sequence that markedly enhances the translation efficiency of mRNA transcripts when it is inserted at the 3'-end of the 5' UTR. This new sequence allows engineering artificial transcripts with very high protein production yields that may be useful for a wide variety of applications, including nucleic acid therapeutics or industrial recombinant protein production.

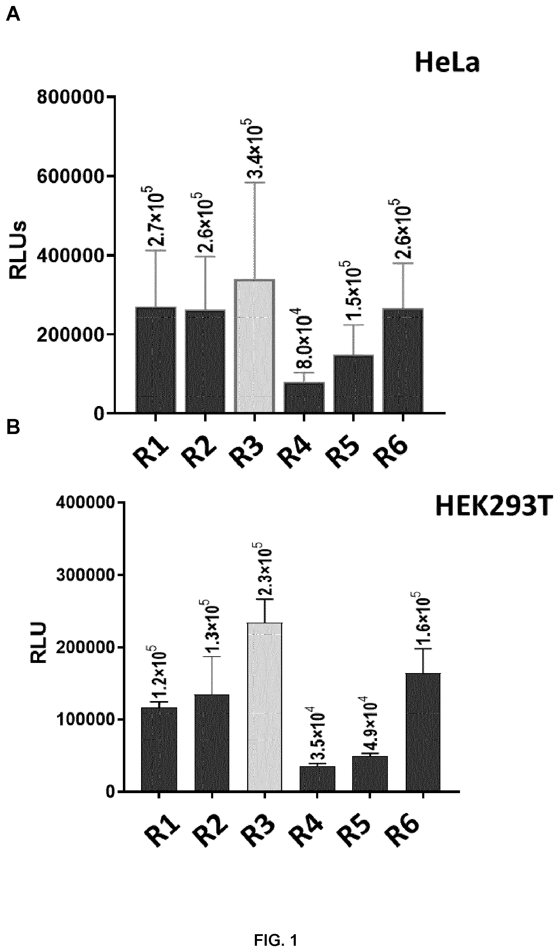

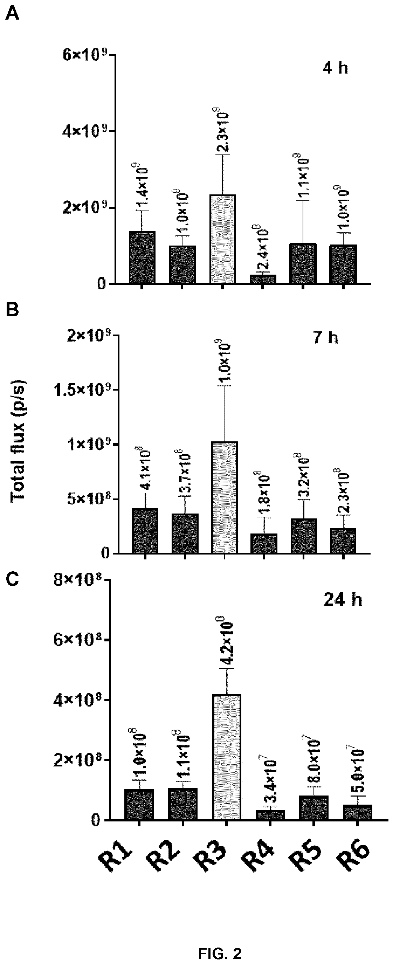

As shown in the examples below, the present inventors found that inserting only one repeat of the sequence 5'-GCCACC-3' at the 3'-end of a transcript's 5'-UTR did not affect its protein production capacity, either *in vitro* or *in vivo* (see Figure 1 and 2, R2 vs R1). However, when two tandem repeats of the said sequence were inserted, a remarkable increase in protein production was observed both *in vitro* (see Figure 1, R3 vs R1) and *in vivo* (see Figure 2, R3 vs R1), which was completely unexpected in view of the complete absence of effect observed when only one repeat was present.

Notably, the synergistic effect provided by the presence of the two tandem repeats allowed for an increase of around 400% in protein expression *in vivo* after 24 hours with respect to transcripts not containing the two tandem repeats (Figure 2, R3 vs R1 or R2), and also transcripts containing the regulatory elements of commercial RNAs (Figure 2, R3 vs R4 or R6) or previously disclosed RNAs (Figure 2, R3 vs R5).

Thus, from the data provided below it is apparent that the polynucleotides provided by the present invention represent an important new tool to overcome the limitations of current artificial RNAs, in particular, their low protein production efficiency. Also, the higher translation efficiency of the polynucleotides of the invention may allow reducing the therapeutic dose of RNA required for genetic vaccination, which may help to reduce the associated secondary effects.

In a first aspect, the invention provides an artificial polynucleotide comprising, in the 5' to 3' direction, a 5' untranslated region (5'-UTR), and an open reading frame (ORF), wherein the 5'-UTR comprises, at its 3'-end, at least two tandem repeats of sequence 5'-GCCNCC-3' operatively linked to the ORF, wherein N is any nucleotide.

In a second aspect, the invention provides a DNA construct comprising a promoter operatively linked to a sequence encoding a polynucleotide as defined in the first aspect.

In a third aspect, the invention provides an expression vector comprising the DNA construct as defined in the second aspect.

In a fourth aspect, the invention provides a cell comprising the polynucleotide as defined in the first aspect, a DNA construct as defined in the second aspect, or an expression vector as defined in the third aspect.

In a fifth aspect, the invention provides a composition comprising a lipid nanoparticle and a polynucleotide as defined in the first aspect, a DNA construct as defined in the second aspect, or an expression vector as defined in the third aspect.

In a sixth aspect, the invention provides a pharmaceutical composition comprising a therapeutically effective amount of a polynucleotide according to the first aspect, a DNA construct according to the second aspect, an expression vector according to the third aspect, or a composition according to the fifth aspect, and at least one pharmaceutically acceptable excipient and/or carrier.

When the polynucleotide of the invention includes an ORF encoding a protein associated with a disease, all the previous listed aspects can be used as a medicament. Thus, in a seventh aspect, the invention provides a polynucleotide according to the first aspect, a DNA construct according to the second aspect, an expression vector according to the third aspect, a cell according to the fourth aspect, a composition according to the fifth aspect, or a pharmaceutical composition according to the sixth aspect, for use in medicine.

In an eighth aspect, the invention provides a polynucleotide according to the first aspect, a DNA construct according to the second aspect, an expression vector according to the third aspect, a cell according to the fourth aspect, a composition according to the fifth aspect, or a pharmaceutical composition according to the sixth aspect, for use in a method of inducing an immune response in a subject.

This aspect can also be formulated as the use of the polynucleotide, the DNA construct, the expression vector, the composition, the cell, or the pharmaceutical composition, for the manufacture of a medicament for inducing an immune response in a subject. This aspect can also be formulated as a method for inducing an immune response in a subject, the method comprising administering a therapeutically effective amount of the polynucleotide, the DNA construct, the expression vector, the composition, the cell, or the pharmaceutical composition of the invention to a subject in need thereof.

In a ninth aspect, the invention provides a polynucleotide according to the first aspect, a DNA construct according to the second aspect, an expression vector according to the third aspect, a cell according to the fourth aspect, a composition according to the fifth aspect, or a pharmaceutical composition according to the sixth aspect, for use in a method for the therapeutic immunization of a subject.

This aspect can also be formulated as the use of the polynucleotide, the DNA construct, the expression vector, the composition, the cell, or the pharmaceutical composition, for the manufacture of a medicament for the therapeutic immunization of a subject. This aspect can also be formulated as a method for the therapeutic immunization of a subject, the method comprising administering a therapeutically effective amount of the polynucleotide, the DNA construct, the expression vector, the composition, the cell, or the pharmaceutical composition of the invention to a subject in need thereof.

In a tenth aspect, the invention provides a polynucleotide according to the first aspect, a DNA construct according to the second aspect, an expression vector according to the third aspect, a cell according to the fourth aspect, a composition according to the fifth aspect, or a pharmaceutical composition according to the sixth aspect, for use as a vaccine or for use in gene therapy.

In an eleventh aspect, the invention provides an *in vitro* method of producing a polypeptide in a cell, the method comprising contacting the cell with a polynucleotide as defined in the first aspect, a DNA construct as defined in the second aspect, an expression vector as defined in the third aspect, a composition as defined in the fifth aspect, or a pharmaceutical composition as defined in the sixth aspect.

In a twelfth aspect, the invention provides a method to increase the translation rate of a polynucleotide comprising in the 5' to 3' direction a 5' untranslated region (5'-UTR) and an open reading frame (ORF), the method comprising the step of inserting at least two tandem repeats of sequence 5'-GCCNCC-3' at the 3'-end of the 5'-UTR, wherein N is any nucleotide.

In a thirteenth aspect, the invention provides a vaccination kit comprising (a) a polynucleotide as defined in the first aspect, a DNA construct as defined in the second aspect, a DNA vector as defined in the third aspect, an expression vector as defined in the fourth aspect, or a composition as defined in the fifth aspect; (b) a pharmaceutically acceptable excipient and/or carrier; (c) optionally an adjuvant; and (d) optionally instructions for its use.

In a fourteenth aspect, the invention provides a process for making the pharmaceutical composition according to the sixth aspect, which comprises the step of mixing the polynucleotide, the DNA construct, the expression vector, or the composition of the invention with at least one pharmaceutically acceptable excipient and/or carrier.

### Brief Description of Drawings

Fig. 1, related to Example 1, is a bar diagram showing Luciferase production levels in (A) HeLa cells and (B) HEK293T cells after transfection with the indicated mRNAs. The structure of the mRNAs R1, R2, R3, R4, R5, and R6 is detailed in Table 2 below. "RLU" represents relative light units.
Fig. 2, related to Example 2, is a bar diagram showing Luciferase production levels in mouse muscle administered with LNPs containing the indicated mRNAs after (A) 4 h, (B) 7h, and (C) 24h. The structure of the mRNAs R1, R2, R3, R4, R5, and R6 is detailed in Table 2 below.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

The term "polynucleotide" is interchangeably used with "nucleic acid" and refers to a polymer of nucleotides, either ribonucleotides or deoxyribonucleotides. A polynucleotide formed by ribonucleotides may be referred to as "RNA polynucleotide", "ribonucleic acid" or simply "RNA"; and a polynucleotide formed by deoxyribonucleotides may be referred to as "DNA polynucleotide", "deoxyribonucleic acid" or simply "DNA". The polynucleotide may be single- or double-stranded, optionally incorporating synthetic, non-natural, or altered nucleotides capable of incorporation into DNA or RNA. "Artificial polynucleotide" refers to a polynucleotide with a sequence that does not occur in nature or that has been altered by human intervention. A "purine nucleotide" is a nucleotide that contains a purine as a nitrogenous base, in particular, adenine or guanine. When sequences of polynucleotides are indicated, they refer to informative molecules and only one strand is shown. Nonetheless, the skilled person in the art will understand that they can be in the form of single or double stranded nucleic acid polymers; for example, an mRNA is single stranded; and a DNA construct is double stranded.

As used herein, the terms "messenger RNA" or "mRNA" or "transcript" refers to any RNA polynucleotide which encodes a polypeptide of interest and which is capable of being translated to produce the encoded polypeptide of interest *in vitro*, *in vivo*, or ex *vivo.* Typically, an mRNA is single-stranded and comprises a 5'-cap structure, a 5'UTR, an ORF, a 3'UTR and a 3' tailing sequence.

As used herein, "untranslated region" or "UTR" of a polynucleotide refers to a region located upstream (i.e., 5') or downstream (i.e., 3') of an open reading frame (ORF) and that is not translated by the ribosome. Accordingly, a "5' untranslated region" or "5'-UTR" refers to a region located upstream (i.e., 5') of the start codon of the ORF, which is transcribed but not translated into an amino acid sequence. The 5'-UTR typically corresponds to the sequence which extends from a nucleotide located immediately 3' to the 5'-cap, to the nucleotide located immediately 5' to the start codon of the ORF. A "3' untranslated region" or "3' UTR" refers to a region located downstream of the stop codon of the ORF, which is transcribed but not translated into an amino acid sequence. The 3'-UTR typically corresponds to the sequence which extends from a nucleotide immediately 3' to the stop codon of the ORF to the nucleotide immediately 5' to the poly(A) sequence.

As used herein, "open reading frame" or "ORF" refers to a sequence of several nucleotide triplets that encodes a polypeptide, that is, that can be translated into a polypeptide sequence. An open reading frame typically contains a start codon, i.e., a combination of three nucleotides coding typically for the amino acid methionine (e.g., ATG or AUG) at its 5' end, and a subsequent region, which usually exhibits a length, which is a multiple of 3 nucleotides. An ORF is typically terminated by a stop-codon (e.g., TAA, TAG, TGA). An open reading frame may also be termed "protein coding region".

A "5'-cap structure" refers to an entity, typically a modified nucleotide entity, which generally closes the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g., m7GpppN, wherein N is the terminal 5' nucleotide of the nucleic acid carrying the 5'-cap, typically the 5'-end of an RNA. A 5'-cap structure includes, without limitation, "cap-0" that refers to a methylated guanosine (on the nitrogen at the seven position, N⁷) added in the 5'-to-5' linkage to the mRNA; "cap-1" that refers to cap-0 with the addition of a methyl group to the 2'-carbon of ribose on the nucleotide (N₁) at the 5'-end of the chain; and "cap-2" that refers to cap-1 with the addition of another 2'-methyl group to the next nucleotide (N₂).

A "3' tailing sequence" refers to a sequence located at the 3'-end of an mRNA that enhances the stability of the mRNA and is typically rich in adenine nucleotides and at least 80 nucleotides in length.

As used herein, "operatively linked" or "operably linked" refers to a linkage of sequences in a way that the expression of the coding sequence is achieved under conditions compatible with the control sequences. Operably linked sequences include both expression control sequences that are contiguous with the coding sequence of interest, and expression control sequences that act in trans or at a distance to control the coding sequence of interest. For example, the at least two tandem repeats of sequence 5'-GCCNCC-3' located at the 3'-end of the 5'-UTR is an expression control sequence operatively linked to the ORF located downstream as it enhances the translation of the ORF. In a particular embodiment, "operatively linked sequences" refers to sequences directly linked.

As used herein, the term "tandem repeats" refers to a sequence occurring in sequential succession. For example, two tandem repeats of sequence 5'-GCCNCC-3' refers to a sequence containing two consecutive units of 5'-GCCNCC-3' (i.e., 5'-GCCNCCGCCNCC-3').

As used herein, a "Kozak sequence" refers to a sequence located within an mRNA that helps ribosomal translation machinery to recognize where the transcript translation should begin. Kozak sequences have the consensus sequence CCRCCAUGG, where R is a purine nucleotide, and the AUG is the start codon of the ORF. A "consensus Kozak sequence" or "strong Kozak sequence", as used herein, refers to a sequence that closely matches the consensus sequence above. A "non-consensus Kozak sequence" or "weak Kozak sequence" refers to a sequence that does not closely match the consensus sequence, particularly at positions +4 and -3 relative to the adenosine (+1) at the 5' end of the start codon.

The term "heterologous" refers to a combination of elements not naturally occurring. For example, a 5'-UTR that is heterologous to an ORF means that the 5'-UTR is not found combined with that ORF in a natural sequence.

In the present invention the term "identical" or "identity" refers to the percentage of positions that are identical in the two sequences when the sequences are optimally aligned. If, in the optimal alignment, a position in a first sequence is occupied by the same nucleotide as the corresponding position in the second sequence, the sequences exhibit identity with respect to that position. The percentage of identity determines the number of identical nucleotides over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) × 100). A gap, i.e., a position in an alignment where a nucleotide is present in one sequence but not in the other, is regarded as a position with non-identical nucleotide and is counted as a compared position.

A number of mathematical algorithms for rapidly obtaining the optimal alignment and calculating identity between two or more sequences are known and incorporated into a number of available software programs. For purposes of the present invention, the sequence identity between two nucleic acid sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

As used herein, "DNA construct" refers to an artificial polynucleotide including a sequence of interest operatively linked to an expression promoter, said promoter controlling expression of the sequence of interest. An "expression vector" refers to a vector used to introduce a specific nucleic acid, typically a DNA construct, into a target cell for expression of the nucleic acid by the cell. Examples of suitable expression promoters and expression vectors include those conventionally used in molecular biology and known to the skilled person.

The term "polypeptide" refers to any peptide or protein comprising two or more amino acids joined to each other by peptide bonds or modified peptide bonds (i.e., peptide isosteres). "Polypeptide" refers to both short chains, commonly referred as peptides, oligopeptides, or oligomers, and to longer chains generally referred to as proteins.

The expression "therapeutically effective amount" as used herein, refers to the amount of a compound that, when administered, is sufficient to prevent the development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and similar considerations. The term "pharmaceutical" encompasses also the concept of "veterinary composition". Thus, they relate to compositions that are therapeutically effective when administered by any desired or applicable route to any animal, including humans.

According to the present invention, the term "antigen" is a compound that can be recognized by immunoglobulin receptors of B cells, or by the T-cell receptor when complexed with MHC. Preferably, an "antigen" is a polypeptide.

The term "nanoparticle" as used herein, refers to a particle with at least two dimensions at the nanoscale, particularly with all three dimensions at the nanoscale, where the nanoscale is in the range of about 1 nm to about 300 nm. Particularly, when the nanoparticle is substantially rod-shaped with a substantially circular cross-section, such as a nanowire or a nanotube, the "nanoparticle" refers to a particle with at least two dimensions at the nanoscale, these two dimensions being the cross-section of the nanoparticle. The term "lipidic nanoparticle" as used herein, refers to a nanoparticle whose external envelope is totally or partially made of lipids. Suitable lipid nanoparticles that can be used in the compositions of the invention are described, for example, in Hassett, K. J. et al., "Optimization of Lipid Nanoparticles for Intramuscular Administration of mRNA Vaccines", 2019, Mol. Ther. Nucleic Acid, vol. 15, pp. 1-11.

As mentioned above, in a first aspect the invention provides an artificial polynucleotide comprising, in the 5' to 3' direction, a 5' untranslated region (5'-UTR) and an open reading frame (ORF), wherein the 5'-UTR comprises at its 3'-end at least two tandem repeats of sequence 5'-GCCNCC-3' operatively linked to the ORF, wherein N is any nucleotide.

In a particular embodiment of the first aspect, optionally in combination with any of the embodiments above or below, the at least two tandem repeats are of sequence 5'-GCCRCC-3', wherein R is a purine nucleotide.

In an embodiment of the first aspect, the artificial polynucleotide comprises, in the 5' to 3' direction, a 5' untranslated region (5'-UTR) and an open reading frame (ORF), wherein the 5'-UTR comprises at its 3'-end at least two tandem repeats of sequence 5'-GCCRCC-3' operatively linked to the ORF, wherein R is a purine nucleotide.

In an embodiment of the first aspect, the polynucleotide comprises, consecutively and in the 5' to 3' direction, a 5' untranslated region (5'-UTR) and an open reading frame (ORF), wherein the 5'-UTR comprises at its 3'-end at least two tandem repeats of the sequence 5'-GCCNCC-3' operatively linked to the ORF, and wherein N is any nucleotide. In a more particular embodiment, the two tandem repeats are of sequence 5'-GCCRCC-3', wherein R is a purine nucleotide.

In an embodiment of the first aspect, the polynucleotide comprises, in the 5' to 3' direction a 5' untranslated region (5'-UTR) and an open reading frame (ORF), wherein the 5'-UTR comprises at its 3'-end at least two tandem repeats of sequence 5'-GCCRCC-3' operatively linked to the ORF, wherein R is an adenine nucleotide or guanine nucleotide.

In an embodiment of the first aspect, the at least two tandem repeats of sequence 5'-GCCNCC-3' or 5'-GCCRCC-3' are directly linked to the ORF (that is, the at least two tandem repeats are located immediately upstream of the first codon of the ORF).

In an embodiment of the first aspect, the 5'-UTR comprises at its 3'-end two tandem repeats of the sequence 5'-GCCNCC-3' operatively linked to the ORF. In an embodiment of the first aspect, the 5'-UTR comprises at its 3'-end three tandem repeats of the sequence 5'-GCCNCC-3' operatively linked to the ORF. In another embodiment, the 5'-UTR comprises at its 3'-end two or three tandem repeats of the sequence 5'-GCCRCC-3' operatively linked to the ORF.

In an embodiment of the first aspect, the at least two tandem repeats of sequence 5'-GCCNCC-3' enhance the translation efficiency of the ORF.

In an embodiment of the first aspect, the 5'-UTR comprises at its 3'-end a sequence selected from the group consisting of SEQ ID NO: 1, SEQ ID NO: 2, SEQ ID NO: 3, SEQ ID NO: 4, SEQ ID NO: 5, and SEQ ID NO: 6. In a more particular embodiment, the 5'-UTR comprises at its 3'-end SEQ ID NO: 3.

In an embodiment of the first aspect, the 5'-UTR comprises at its 3'-end a sequence selected from the group consisting of SEQ ID NO: 1, SEQ ID NO: 2, SEQ ID NO: 3, SEQ ID NO: 4, SEQ ID NO: 5, and SEQ ID NO: 6, or a variant thereof at least 75%, at least 76%, at least 77%, at least 78%, at least 79%, at least 80%, at least 81%, at least 82%, at least 83%, at least 84%, at least 85%, at least 86%, at least 87%, at least 88%, at least 88.5%, at least 89%, at least 89.5%, at least 90%, at least 90.5%, at least 91%, at least 91.5%, at least 92%, at least 92.5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, at least 99.5%, or at least 99.9% identical to SEQ ID NO: 1, SEQ ID NO: 2, SEQ ID NO: 3, SEQ ID NO: 4, SEQ ID NO: 5, and SEQ ID NO: 6, particularly, wherein the variant thereof substantially maintains or improves the translation enhancing effect of SEQ ID NO: 1, SEQ ID NO: 2, SEQ ID NO: 3, SEQ ID NO: 4, SEQ ID NO: 5, or SEQ ID NO: 6.

Polynucleotide sequence variants are well understood to those of skill in the art and can involve sequence modifications including deletions, insertions, or changes of nucleotides.

In a particular embodiment of the first aspect, the at least two tandem repeats of sequence 5'-GCCNCC-3' form a sequence selected from the group consisting of SEQ ID NO: 1, SEQ ID NO: 2, SEQ ID NO: 3, SEQ ID NO: 4, SEQ ID NO: 5, and SEQ ID NO: 6; particularly SEQ ID NO: 3.

In an embodiment of the first aspect, the 5'-UTR comprises or consists of, in the 5' to 3' direction, a sequence from a 5'-UTR of a transcript of a gene linked to at least two tandem repeats of sequence 5'-GCCNCC-3'. In an embodiment of the first aspect, the 5'-UTR comprises or consists of, in the 5' to 3' direction, a sequence from a 5'-UTR of a transcript of a gene linked to two tandem repeats of sequence 5'-GCCNCC-3'. In another embodiment, the 5'-UTR comprises or consists of, in the 5' to 3' direction, a sequence derived from a 5'-UTR of a transcript of a gene linked to the at least two tandem repeats of sequence 5'-GCCNCC-3'. In another embodiment, the 5'-UTR comprises or consists of, in the 5' to 3' direction, the 5'-UTR of a transcript of a gene directly linked to at least two tandem repeats of sequence 5'-GCCNCC-3'. In a more particular embodiment, the at least two tandem repeats are of sequence 5'-GCCRCC-3'. In a more particular embodiment, the gene is a naturally occurring gene. In an even more particular embodiment, the gene is a mammalian gene or a human gene.

In another particular embodiment of the first aspect, the 5'-UTR comprises or consists of, in the 5' to 3' direction, a sequence from a 5'-UTR of a transcript of a gene linked to at least two tandem repeats of sequence 5'-GCCNCC-3', wherein the sequence from a 5'-UTR of a transcript of a gene comprises a Kozak sequence, particularly a non-consensus Kozak sequence. Thus, the Kozak sequence is directly linked to the tandem repeats of sequence 5'-GCCNCC-3'. In another particular embodiment of the first aspect, the 5'-UTR comprises or consists of, in the 5' to 3' direction, a sequence from a 5'-UTR of a transcript of a gene linked to two tandem repeats of sequence 5'-GCCNCC-3', wherein the sequence from a 5'-UTR of a transcript of a gene comprises a Kozak sequence, particularly a non-consensus Kozak sequence. Thus, the polynucleotide may comprise, consecutively and in the 5' to 3' direction, the nucleotides of the Kozak sequence that are upstream of the start codon, the (at least) two tandem repeats of sequence 5'-GCCNCC-3', and then the start codon. In an even more particular embodiment, the two tandem repeats are of sequence 5'-GCCRCC-3'.

In a particular embodiment of the first aspect, the 5'-UTR comprises or consists of, in the 5' to 3' direction, a sequence from a 5'-UTR of a transcript of a gene linked to at least two tandem repeats of sequence 5'-GCCNCC-3', wherein the gene is selected from the group consisting of apolipoprotein A2 (APOA2), hemoglobin subunit beta (HBB), and pre T cell antigen receptor alpha (PTCRA). In a more particular embodiment, the gene is a human gene selected from the group consisting of apolipoprotein A2 (APOA2) (NCBI Gene ID: 336, updated on 7-Aug-2022), hemoglobin subunit beta (HBB) (NCBI Gene ID: 3043, updated on 5-Aug-2022), and pre T cell antigen receptor alpha (PTCRA) (Gene ID: 171558, updated on 5-Aug-2022). In a more particular embodiment, the at least two tandem repeats are of sequence 5'-GCCRCC-3'.

In a particular embodiment of the first aspect, the 5'-UTR comprises or consists of a sequence selected from the group consisting of SEQ ID NO: 9, SEQ ID NO: 12, and SEQ ID NO: 15, or a variant thereof at least 75%, at least 76%, at least 77%, at least 78%, at least 79%, at least 80%, at least 81%, at least 82%, at least 83%, at least 84%, at least 85%, at least 86%, at least 87%, at least 88%, at least 88.5%, at least 89%, at least 89.5%, at least 90%, at least 90.5%, at least 91%, at least 91.5%, at least 92%, at least 92.5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, at least 99.5%, or at least 99.9% identical to SEQ ID NO: 9, SEQ ID NO: 12, or SEQ ID NO: 15, particularly, wherein the variant thereof substantially maintains or improves the translation enhancing effect of SEQ ID NO: 9, SEQ ID NO: 12, or SEQ ID NO: 15.

In a particular embodiment of the first aspect, the polynucleotide further comprises one or more of a 5'-cap structure; a 3' untranslated region (3'-UTR); and a 3' tailing sequence.

In a particular embodiment of the first aspect, the polynucleotide comprises or consists of, in the 5' to 3' direction, (i) a 5'-cap structure; (ii) a 5' untranslated region (5'-UTR), (iii) an open reading frame (ORF); (iv) a 3' untranslated region (3'-UTR); and (v) a 3' tailing sequence.

In a particular embodiment of the first aspect, the polynucleotide is an RNA polynucleotide, particularly a messenger RNA (mRNA).

In a particular embodiment of the first aspect, the 5' UTR is heterologous to the ORF and/or the 3' UTR.

In a particular embodiment of the first aspect, the 3'-UTR comprises or consists of a sequence from a 3'-UTR of a transcript of a gene, particularly a mammalian gene or a human gene.

In a particular embodiment of the first aspect, the 3'-UTR comprises or consists of at least two tandem repeats of a sequence from a 3'-UTR of a transcript of a gene, particularly two tandem repeats of a sequence from a 3'-UTR of a transcript of a gene. Particularly, the 3'-UTR comprises or consists of a sequence or two tandem repeats of a sequence from a 3'-UTR of a transcript of a gene, wherein the gene is selected from the group consisting of HBB, PTCA, and APOA2.

In a particular embodiment of the first aspect, the 3'-UTR comprises or consists of a 3'-UTR of a transcript of a gene, particularly a mammalian gene or a human gene. More particularly, the 3'-UTR comprises or consists of two tandem repeats of a 3'-UTR of a transcript of a gene, particularly, wherein the gene is selected from the group consisting of HBB, PTCA, and APOA2.

In an embodiment of the first aspect, the 3'-UTR comprises or consists of a sequence selected from the group consisting of SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26, or a variant thereof at least 75%, at least 76%, at least 77%, at least 78%, at least 79%, at least 80%, at least 81%, at least 82%, at least 83%, at least 84%, at least 85%, at least 86%, at least 87%, at least 88%, at least 88.5%, at least 89%, at least 89.5%, at least 90%, at least 90.5%, at least 91%, at least 91.5%, at least 92%, at least 92.5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, at least 99.5%, or at least 99.9% identical to SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 or SEQ ID NO: 26.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 9, and the 3'-UTR comprises or consists of sequence SEQ ID NO: 18.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 9 and the 3'-UTR comprises or consists of sequence SEQ ID NO: 19.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 9 and the 3'-UTR comprises or consists of sequence SEQ ID NO: 20.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 9 and the 3'-UTR comprises or consists of sequence SEQ ID NO: 21.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 9 and the 3'-UTR comprises or consists of sequence SEQ ID NO: 22.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 9 and the 3'-UTR comprises or consists of sequence SEQ ID NO: 23.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 15 and the 3'-UTR comprises or consists of sequence SEQ ID NO: 18.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 15 and the 3'-UTR comprises or consists of sequence SEQ ID NO: 19.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 15 and the 3'-UTR comprises or consists of sequence SEQ ID NO: 20.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 15 and the 3'-UTR comprises or consists of sequence SEQ ID NO: 21.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 15 and the 3'-UTR comprises or consists of sequence SEQ ID NO: 22.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 15 and the 3'-UTR comprises or consists of sequence SEQ ID NO: 23.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 12 and the 3'-UTR comprises or consists of sequence SEQ ID NO: 18.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 12 and the 3'-UTR comprises or consists of sequence SEQ ID NO: 19.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 12 and the 3'-UTR comprises or consists of sequence SEQ ID NO: 20.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 12 and the 3'-UTR comprises or consists of sequence SEQ ID NO: 21.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 12 and the 3'-UTR comprises or consists of sequence SEQ ID NO: 22.

In an embodiment of the first aspect, the polynucleotide comprises, in the 5' to 3' direction, a 5'-UTR, an ORF, and a 3'-UTR, wherein the 5'-UTR comprises or consists of sequence SEQ ID NO: 12 and the 3'-UTR comprises or consists of sequence SEQ ID NO: 23.

In an embodiment of the first aspect, the ORF encodes a polypeptide, particularly, wherein the polypeptide is an antigen. More particularly, the antigen is selected from the group consisting of a viral protein, a bacterial protein and a tumour-associated antigen. In a particular embodiment of the first aspect, the polypeptide is an antibody or a fragment thereof. In a more particular embodiment, the antibody or a fragment thereof is a therapeutic antibody or a fragment thereof.

In an embodiment of the first aspect, the 5'-cap structure is selected from the group consisting of cap-0, cap-1, cap-2, ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine. In a more particular embodiment, the 5'-cap structure is cap-1.

In an embodiment of the first aspect, the 3' tailing sequence is a poly(A) region, particularly the poly(A) region is of at least 80 nucleotides, of at least 90 nucleotides, or of at least 100 nucleotides in length. In a more particular embodiment, the 3' tailing sequence comprises or consists of sequence SEQ ID NO: 39, or a variant thereof at least 75%, at least 76%, at least 77%, at least 78%, at least 79%, at least 80%, at least 81%, at least 82%, at least 83%, at least 84%, at least 85%, at least 86%, at least 87%, at least 88%, at least 88.5%, at least 89%, at least 89.5%, at least 90%, at least 90.5%, at least 91%, at least 91.5%, at least 92%, at least 92.5%, at least 93%, at least 93.5%, at least 94%, at least 94.5%, at least 95%, at least 95.5%, at least 96%, at least 96.5%, at least 97%, at least 97.5%, at least 98%, at least 98.5%, at least 99%, at least 99.5%, or at least 99.9% identical to SEQ ID NO: 39.

In an embodiment of the first aspect, the polynucleotide comprises at least one of the following: - a 5' untranslated region (5'-UTR) of sequence SEQ ID NO: 9, SEQ ID NO: 12, or SEQ ID NO: 15; a 3' untranslated region (3'-UTR) of sequence SEQ ID NO: 18, SEQ ID NO: 19, SEQ ID NO: 20, SEQ ID NO: 21, SEQ ID NO: 22, or SEQ ID NO: 23; and a 3' tailing sequence of sequence SEQ ID NO: 39.

In an embodiment of the first aspect, the polynucleotide comprises at least one of the following: - a 5' untranslated region (5'-UTR) of sequence SEQ ID NO: 9; a 3' untranslated region (3'-UTR) of sequence SEQ ID NO: 19; and a 3' tailing sequence of sequence SEQ ID NO: 39.

In an embodiment of the first aspect, the polynucleotide comprises: - a 5' untranslated region (5'-UTR) of sequence SEQ ID NO: 9; a 3' untranslated region (3'-UTR) of sequence SEQ ID NO: 19; and a 3' tailing sequence of sequence SEQ ID NO: 39.

In an embodiment of the first aspect, the polynucleotide comprises or consists of in the 5' to 3' direction (i) a 5'-cap1 structure; (ii) a 5' untranslated region (5'-UTR), particularly of sequence SEQ ID NO: 9, (iii) an open reading frame (ORF); particularly encoding a polypeptide; (iv) a 3' untranslated region (3'-UTR), particularly of sequence SEQ ID NO: 19; and (v) a 3' tailing sequence, particularly of sequence SEQ ID NO: 39.

In an embodiment of the first aspect, the polynucleotide comprises at least one chemical modification. In a particular embodiment, the chemical modification is selected from the group consisting of pseudouridine, N1-methylpseudouridine (also referred to as 1-methylpseudouridine or m1Ψ), N6-methyladenosine (also referred to as m6A), 2-thiouridine (also referred to as s2U), 4'-thiouridine, 5-methylcytosine (also referred to 5mC), 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-Thio-I-methyl-pseudouridine, 4-thio-pseudouridine, 5-aza-uridine, dihydropseudouridine, 5-methyluridine (also referred to as m5U), 5-methoxyuridine, 2'-O-methyl uridine, and combinations thereof. In an even more particular embodiment, the chemical modification is N1-methylpseudouridine, 5-methoxyuridine or a combination thereof; particularly the chemical modification is N1-methylpseudouridine.

In a particular embodiment of the first aspect, the polynucleotide is partially modified with of N1-methylpseudouridine. In another particular embodiment, the polynucleotide is partially modified with at least 10%, at least 20%, at least 30%, at least 40%, at least 50%, at least 60%, at least 70%, at least 80%, at least 90%, or at least 99%, of N1-methylpseudouridine. In another particular embodiment, the polynucleotide is fully modified with N1-methylpseudouridine, 5-methoxyuridine or a combination thereof. In a more particular embodiment, the polynucleotide is fully modified with N1-methylpseudouridine.

In a particular embodiment of the first aspect, the polynucleotide is an isolated artificial polynucleotide.

As above disclosed, in a second aspect the invention provides a DNA construct comprising a promoter operatively linked to a sequence encoding the polynucleotide of the first aspect. That is, this aspect provides a DNA construct the transcription of which produces a polynucleotide as defined in the first aspect.

All the embodiments of the polynucleotide of the first aspect are also meant to apply to the DNA construct of the second aspect.

In an embodiment of the second aspect, the polynucleotide is an RNA polynucleotide, particularly an mRNA.

The skilled person knows how to produce the polynucleotide, the DNA construct, or the expression vector of the invention by routine methods well known in the art, for example, by chemical synthesis or by molecular biology techniques, without exercising any inventive skill.

As above indicated, the invention provides in a fifth aspect a composition comprising a lipid nanoparticle and a polynucleotide as defined in the first aspect, a DNA construct as defined in the second aspect, or an expression vector as defined in the third aspect. That is, the composition comprises a lipid nanoparticle encapsulating a polynucleotide as defined in the first aspect, a DNA construct as defined in the second aspect, or an expression vector as defined in the third aspect.

Methods for the synthesis of the compositions formed by the lipid nanoparticle encapsulating the polynucleotide, DNA construct or expression vector are well-known by the skilled person in the art and duly established in the protocols for molecular biology. Particular conditions are indicated in the examples. The skilled in the art would know, depending on the intended use of the composition, which lipid nanoparticle to use to encapsulate the polynucleotide, the DNA construct, or the expression vector.

In an embodiment of the fifth aspect, the lipid nanoparticle comprises at least one selected from the group consisting of PEG-modified lipid, a non-cationic lipid, a sterol, and an ionizable cationic lipid. In an embodiment of the fifth aspect, the lipid nanoparticle comprises or consists of PEG-modified lipid, a non-cationic lipid, a sterol, and an ionizable cationic lipid. In a more particular embodiment, the lipid nanoparticle comprises at least one of SM-102, distearolyphosphatidycholine (DSPC), Cholesterol, and DMG-PEG2000. In a more particular embodiment, the lipid nanoparticle comprises or consists of SM-102, DSPC, Cholesterol, and DMG-PEG2000.

In a particular embodiment of the fifth aspect, the lipid nanoparticle is ionizable or electrically charged.

In a particular embodiment of the fifth aspect, the composition comprises the polynucleotide, the DNA construct or the expression vector of the invention encapsulated in a lipid nanoparticle.

In a particular embodiment of the fifth aspect, the composition is administered in the form of a pharmaceutical composition.

As above indicated, the invention provides in a sixth aspect a pharmaceutical composition comprising a therapeutically effective amount of a polynucleotide, a DNA construct, an expression vector, or a composition of the invention, and at least one pharmaceutically acceptable excipient and/or carrier.

The expression "pharmaceutically acceptable excipient and/or carrier" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as colouring agents, coating agents, sweetening, and flavouring agents can be present in the composition, according to the judgment of the formulator.

The polynucleotides or compositions as described herein can be used in vaccine therapy, in the enhancement of the efficacy of a conventional vaccine and/or as a novel vaccine form for use against infectious pathogens, such as viruses, bacteria, fungi, protozoa, prions, and helminths (worms); or for use in treating diseases such as cancer.

In an embodiment of the sixth aspect, the pharmaceutical composition is a vaccine. In a more particular embodiment, the pharmaceutical composition is a vaccine and further comprises an adjuvant. The skilled person would know, based on its common general knowledge, which excipients, carriers, and adjuvants to include in the vaccine depending on the intended use.

In a further embodiment, the polynucleotide, the DNA construct, the expression vector, the cell, the composition, or the pharmaceutical composition of the invention is for use in the prevention of COVID-19.

In an embodiment of the eighth, ninth, and tenth aspect, the polynucleotide, the DNA construct, the expression vector, the composition, or the pharmaceutical composition is administered orally, intranasally, intravenously, intraperitoneally, intramuscularly, intradermally, subcutaneously, topically, or by intra-articular administration.

As above indicated, the invention also provides in an eleventh aspect an *in vitro* method of producing a polypeptide in a cell, the method comprising contacting the cell with a polynucleotide, a DNA construct, an expression vector, a composition, or a pharmaceutical composition of the invention; particularly, under suitable conditions. Those skilled in the art will be able to determine operative and optimal conditions for producing the polypeptide by employing routine experimentation.

The invention also provides in a twelfth aspect a method to increase the translation rate of a polynucleotide. The skilled person knows how to insert the tandem repeat sequences into the sequence of the polynucleotide using conventional molecular biology techniques.

In a particular embodiment of the twelfth aspect, the method comprises the step of inserting at least two tandem repeats of sequence 5'-GCCRCC-3' at the 3'-end of the 5'-UTR, wherein R is a purine nucleotide. Particularly, the at least two tandem repeats of sequence 5'-GCCRCC-3' are located immediately upstream of the start codon of the ORF.

In a particular embodiment of the twelfth aspect, the method comprises the step of inserting two tandem repeats of sequence 5'-GCCRCC-3' at the 3'-end of the 5'-UTR, particularly two tandem repeats of sequence 5'-GCCACC-3' at the 3'-end of the 5'-UTR.

All embodiments of the first aspect regarding the polynucleotide sequence are also meant to apply to this twelfth aspect.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

Sequences of the present invention are disclosed in Table 1 below:

**Table 1**

| **SEQ ID** | **Sequence** |
|---|---|
| **NO:** | |
| 1 | GCCNCCGCCNCC |
| 2 | GCCRCCGCCRCC |
| 3 | GCCACCGCCACC |
| 4 | GCCACCGCCGCC |
| 5 | GCCGCCGCCACC |
| 6 | GCCGCCGCCGCC |
| 7 | AGGCACAGACACCAAGGACAGAGACGCUGGCUAGGCCGCCCUCCCCACU GUUACCAAC |
| 8 | AGGCACAGACACCAAGGACAGAGACGCUGGCUAGGCCGCCCUCCCCACU GUUACCAACGCCACC |
| 9 | AGGCACAGACACCAAGGACAGAGACGCUGGCUAGGCCGCCCUCCCCACU GUUACCAACGCCACCGCCACC |
| 10 | ACAUUUGCUUCUGACACAACUGUGUUCACUAGCAACCUCAAACAGACAC C |
| 11 | ACAUUUGCUUCUGACACAACUGUGUUCACUAGCAACCUCAAACAGACAC CGCCACC |
| 12 | ACAUUUGCUUCUGACACAACUGUGUUCACUAGCAACCUCAAACAGACAC CGCCACCGCCACC |
| 13 | UAGAAGGCAGUCUUGUGGGUGCCUCCUCCCCCAGCCGCAACUCAGGUC UGCAGCUGGGUCCUGCCUCCUUCCGAGUGGGCC |
| 14 | UAGAAGGCAGUCUUGUGGGUGCCUCCUCCCCCAGCCGCAACUCAGGUC UGCAGCUGGGUCCUGCCUCCUUCCGAGUGGGCCGCCACC |
| 15 | UAGAAGGCAGUCUUGUGGGUGCCUCCUCCCCCAGCCGCAACUCAGGUC UGCAGCUGGGUCCUGCCUCCUUCCGAGUGGGCCGCCACCGCCACC |
| 16 | AAAUAAGAGAGAAAAGAAGAGUAAGAAGAAAUAUAAGACCCCGGCGCCGC CACC |
| 17 | AAAUAAGAGAGAAAAGAAGAGUAAGAAGAAAUAUAAGAGCCACC |
| 18 | GCUCGCUUUCUUGCUGUCCAAUUUCUAUUAAAGGUUCCUUUGUUCCCUA AGUCCAACUACUAAACUGGGGGAUAUUAUGAAGGGCCUUGAGCAUCUGG AUUCUGCCUAAUAAAAAACAUUUAUUUUCAUUGC |
| 19 | GCUCGCUUUCUUGCUGUCCAAUUUCUAUUAAAGGUUCCUUUGUUCCCUA AGUCCAACUACUAAACUGGGGGAUAUUAUGAAGGGCCUUGAGCAUCUGG AUUCUGCCUAAUAAAAAACAUUUAUUUUCAUUGCGCUCGCUUUCUUGCU GUCCAAUUUCUAUUAAAGGUUCCUUUGUUCCCUAAGUCCAACUACUAAA CUGGGGGAUAUUAUGAAGGGCCUUGAGCAUCUGGAUUCUGCCUAAUAAA AAACAUUUAUUUUCAUUGC |
| 20 | GGGCAGGGCUCUACCUCCCCUGCGUCACACUGUGUGAGGCUGUGUCUC UGCCAUCCAAAAGGGGGCCCCUUGAGAAUGGUGAUCCACCCAGUUACAG GGGCAUUUAGGGAGCAGAUGACUGAGAACAUUAAAAAAGAACUUAAAUG ACACAGC |
| 21 | GGGCAGGGCUCUACCUCCCCUGCGUCACACUGUGUGAGGCUGUGUCUC UGCCAUCCAAAAGGGGGCCCCUUGAGAAUGGUGAUCCACCCAGUUACAG GGGCAUUUAGGGAGCAGAUGACUGAGAACAUUAAAAAAGAACUUAAAUG ACACAGCGGGCAGGGCUCUACCUCCCCUGCGUCACACUGUGUGAGGCU GUGUCUCUGCCAUCCAAAAGGGGGCCCCUUGAGAAUGGUGAUCCACCC AGUUACAGGGGCAUUUAGGGAGCAGAUGACUGAGAACAUUAAAAAAGAA CUUAAAUGACACAGC |
| 22 | AGUGUCCAGACCAUUGUCUUCCAACCCCAGCUGGCCUCUAGAACACCCA CUGGCCAGUCCUAGAGCUCCUGUCCCUACCCACUCUUUGCUACAAUAAA UGCUGAAUGAAUCCA |
| 23 | AGUGUCCAGACCAUUGUCUUCCAACCCCAGCUGGCCUCUAGAACACCCA CUGGCCAGUCCUAGAGCUCCUGUCCCUACCCACUCUUUGCUACAAUAAA UGCUGAAUGAAUCCAAGUGUCCAGACCAUUGUCUUCCAACCCCAGCUGG CCUCUAGAACACCCACUGGCCAGUCCUAGAGCUCCUGUCCCUACCCACU CUUUGCUACAAUAAAUGCUGAAUGAAUCCA |
| 24 | GCUGGAGCCUCGGUGGCCUAGCUUCUUGCCCCUUGGGCCUCCCCCCAG CCCCUCCUCCCCUUCCUGCACCCGUACCCCCGUGGUCUUUGAAUAAAGU CUGAGUGGGCGGC |
| 25 | GCUGGAGCCUCGGUGGCCAUGCUUCUUGCCCCUUGGGCCUCCCCCCAG CCCCUCCUCCCCUUCCUGCACCCGUACCCCCGUGGUCUUUGAAUAAAGU CUGAGUGGGCGG |
| 26 | UUAAUUAAGCUGCCUUCUGCGGGGCUUGCCUUCUGGCCAUGCCCUUCU UCUCUCCCUUGCACCUGUACCUCUUGGUCUUUGAAUAAAGCCUGAGUAG GAAG |
| 39 | AAAAAAAAAAAAAAAAAAAAAAAAAAAAAAAGAAAAAAAAAAAAAAAAAAAAAA AAAAAAAAAAAAAAAAAAAAAAAAAAAAAAAAAAAAAAAAAAAAAAAAA |

The following examples are provided by way of illustration, and they are not intended to be limiting of the present invention. Furthermore, the present invention covers all possible combinations of particular and preferred embodiments described herein.

### Examples

### Example 1: protein expression in cells transfected with the RNA of the invention

### In vitro transcription of RNA

The plasmids used in the experiments are listed in Table 2 below:

**Table 2**

| **Plasmid** | **mRNA** | **5'-UTR** | **Repeats of GCCACC** | **3'-UTR** |
|---|---|---|---|---|
| P1 (SEQ ID No: 33) | R1 (SEQ ID No: 27) | APOA2 (SEQ ID No: 7) | 0 | 2*HBB (SEQ ID No: 19) |
| P2 (SEQ ID No: 34) | R2 (SEQ ID No: 28) | APOA2 + GCCACC (SEQ ID No: 8) | 1 | 2*HBB (SEQ ID No: 19) |
| P3 (SEQ ID No: 35) | R3 (SEQ ID No: 29) | APOA2 + GCCACCGCCACC (SEQ ID No: 9) | 2 | 2*HBB (SEQ ID No: 19) |
| P4 (SEQ ID No: 36) | R4 (SEQ ID No: 30) | MOD01 (SEQ ID No: 16) | 1 | MOD01 (SEQ ID No: 24) |
| P5 (SEQ ID No: 37) | R5 (SEQ ID No: 31) | MOD02 (SEQ ID No: 17) | 1 | HBA01 (SEQ ID No: 25) |
| P6 (SEQ ID No: 38) | R6 (SEQ ID No: 32) | MOD02 (SEQ ID No: 17) | 1 | mHba01 (SEQ ID No: 26) |

All plasmids encoded transcripts containing the Firefly Luciferase coding sequence under the control of different UTRs as indicated in Table 2. Plasmid P2 was generated by inserting the sequence 5'-GCCACC-3' at the 3'-end of the 5'-UTR of P1, and plasmid P3 was generated by inserting two tandem repeats of the sequence 5'-GCCACC-3' at the 3'-end of the 5'-UTR of P1. Plasmids P4-P6 encoded transcripts with regulatory elements (i.e., UTRs) of commercially available RNAs, and were used for comparative purposes together with P1 and P2.

1ug of each RNAse-free plasmid was digested with BspQI restriction enzyme for P1, P2, P3 and P5 and Notl for P4 and P6 plasmids. The restriction enzyme cleaved the plasmid right after the segment to be transcribed. The plasmid linearization reaction was purified according to the Wizard^{®} SV Gel and PCR Clean-Up (Promega A7270) purification kit protocol following manufacturer's instructions. The recovery yield of plasmid linearization was in all cases at least 50 %.

The purified linear DNA was subsequently used for RNA production by in vitro transcription using T7 RNA polymerase following manufacturer's instructions. Briefly, transcriptions were performed at 37 °C for 3 hours using the following reagents:
- Template DNA (50 µg/mL)
- T7 polymerase (5.000 U/mL; HONGENE^{®} ON-004)
- RNase inhibitor (1.000 U/mL, HONGENE^{®} ON-039)
- Inorganic Pyrophosphatase (2 U/mL, HONGENE^{®} ON-025)
- ATP (5 µg/mL, HONGENE^{®} R1331)
- GTP (5 µg/mL, HONGENE^{®} R2331)
- CTP (5 µg/mL, HONGENE^{®} R3331)
- N1-methylpseudouridine (5 µg/mL, HONGENE^{®} R5-027)
- CleanCap^{®} AG (4 µg/mL, TRILINK^{®} N-7113-10)

Purification of the generated transcripts was performed by DNase I incubation (NEB^{®} M0303L) following manufacturer's instructions followed by LiCI precipitation and 75% ethanol wash. The concentrations of RNA reconstituted in sodium citrate buffer were determined by measuring the optical density at 260 nm. The yield of RNA production was at least 80 µg of RNA per 1 µg of linearized plasmid DNA.

All RNA samples were analyzed by denaturing agarose gel electrophoresis for quality assurance. RNA was aliquoted and stored at -80 °C until use. Each RNA type was synthesized in at least 2 independently performed transcription experiments and all experiments were performed with at least two different batches of mRNA.

As indicated in Table 2 above, the in vitro transcription of P1 generated the transcript R1; P2 generated the transcript R2; P3 generated the transcript R3; P4 generated the transcript R4; P5 generated the transcript R5; and P6 generated the transcript R6.

### Cell transfection

The day before transfection, HeLa (ACC57) or HEK293T (CRL-3216) cells were seeded into 96-well plates at a density of 1 × 10⁴ cells/well. Culture media used for both cells was DMEM high glucose (Merk D6429) supplemented with Fetal Bovine Serum 10% (Sigma F7524), Antibiotic Antimycotic Solution 1% (Sigma A5955) and Glutamax 2 mM (Fisher 35050038).

For transfection, the corresponding culture media was replaced with 90 µL of fresh one. Then, 10 µL of a mixture of an mRNA R1-R6 produced as above indicated (0.1 ug/well) and Lipofectamine^{™} MessengerMAX reagent (Invitrogen 15397974; 0.2 µL/well) preincubated in OptiMEM media, was added to the cell culture. Cells with mRNA-Lipofectamine MessengerMAX mixture were incubated for 24h at 37 °C, in a 5% CO₂ atmosphere for further analyses.

### Firefly Luciferase activity quantification

Transfected cells were lysed in 100 µL of PBS-Triton 0.1 %, and 98 µL transferred to an opaque 96 well white plate. Then, buffered d-Luciferin (GoldBio LUCK-100) (100mM Tris-HCI pH 7.8, 5mM MgCL₂, 250µM CoA, 150µM ATP) was added in 102 µL at a final concentration of 150 µg/mL to the white 96 well plate. Luminescence was measured after 5 minutes of incubation at room temperature in a FLUOstar Omega plate reader following manufacturer's instructions.

As shown in Figure 1 A and B, the different mRNAs produced luciferase at different rates depending on the combination of 5-'UTR and 3'-UTR sequences flanking the coding region. The addition of one repeat of the sequence 5'-GCCACC-3' right before the ORF in R1 (i.e., R2) did not improve protein production. However, the addition of two 5'-GCCACC-3' sequences in tandem right before the ORF in R1 (i.e., R3) dramatically improved protein production both in HeLa and HEK293 cells, reaching the highest production levels of all mRNAs tested.

These results clearly suggest that the presence of at least two 5'-GCCACC-3' sequences in tandem at the 3'-end of the 5'-UTR synergistically enhances the translation rate of the mRNA thereby allowing higher protein production levels.

### Example 2: protein expression in mice muscle administered with the RNA of the invention

### RNA encapsulation into lipid nanoparticles

For *in vivo* administration, the mRNAs R1-R6 produced as above indicated were encapsulated into lipid nanoparticles (LNPs) as described in Hassett, K. J. et al., "Optimization of Lipid Nanoparticles for Intramuscular Administration of mRNA Vaccines", 2019, Mol. Ther. Nucleic Acid, vol. 15, pp. 1-11. Briefly, the purified capped mRNAs were first diluted in sodium citrate buffer at pH=4 at a final concentration of 266 µg/ml. Separately, lipids SM-102 (BOCSI 2089251-47-6): DSPC (Merk 850365P): Cholesterol (Sigma C3045): DMG-PEG2000 (Cayman 33945-1)) were dissolved into ethanol at the respective molar ratios of 50:10:38.5:1.5, and with an N:P ratio of 5.5:1.

The aqueous solution was then carefully added above the ethanol solution, and the resulting solution was homogenized by pipetting up and down for 4-5 times. The resulting LNPs were immediately diluted 1:1 with Tris buffer and dialyzed overnight against Tris buffer containing 15% sucrose. The resulting LNP solution was then collected, and encapsulated mRNA was assessed by Quant-IT^{®} Ribogreen (Invitrogen R11490) following the manufacturer's instructions.

The LNP solution was then adjusted to a final concentration of mRNA of 100 µg/ml. Size distribution, polydispersity and Z-potential were measured by dynamic light scattering (DLS). Typical values obtained for these parameters ranged as follows: size distribution, 90-120 nm; polydispersity, 0.08-1.5; Z-potential, -10 - +10 mV. RNA encapsulation was assessed by Quant-IT^{®} Ribogreen following the manufacturer's instructions, typically obtaining an encapsulation efficiency of around 80%-95% for all mRNAs.

Finally, the LNP solution was passed through a 0.22 mm filter and the LNPs were stored at -80 °C until required.

### Administration of the mRNA (LNP) to mice

Female BALB/c mice (Charles River Laboratories), 8-10-week-old, weighting 18-23 g were acclimatized to new conditions upon arrival to experimental facilities for 3-7 days. Housing conditions were room temperature 20-24 °C, humidity 50-70 %, and light intensity 60 lux with a light-dark cycle of 12 hours.

For Firefly Luciferase activity measurement in mice, LNPs produced as indicated above and containing 5 µg of the indicated mRNA in 50 µl final volume, were injected intramuscularly.

From 4 to 72 hours after RNA-LPN inoculation mice were anesthetized by inhalation with 4% of Isoflurane using a vaporizer. The maintenance of the anesthesia was performed at 1.5% of Isoflurane. Then, D-luciferin (Quimigen, Ref: 12507) was injected intraperitoneally at 150 mg/kg, normally -200 µL of the stock at 15 mg/mL in PBS for a 20 g mouse. Luciferase images were acquired 10 minutes after luciferin inoculation using the IVIS Lumina XRMS Imaging System following manufacturer's instructions.

As shown in Figure 2, the presence of two 5'-GCCACC-3' sequences in tandem (R3) provided a dramatic increase in protein production *in vivo* compared to LNPs with mRNAs containing only one repeat or none.

These results demonstrated that the presence of two 5'-GCCACC-3' sequences in tandem in polynucleotides for producing proteins greatly enhances their expression capacity, which may improve the therapeutic efficiency of known and future protein expression vectors.

### The invention comprises the following embodiments:

1. An artificial polynucleotide comprising in the 5' to 3' direction:
   - a 5' untranslated region (5'-UTR), and
   - an open reading frame (ORF),
   wherein the 5'-UTR comprises, at its 3'-end, at least two tandem repeats of sequence 5'-GCCNCC-3' operatively linked to the ORF, and wherein N is any nucleotide.
2. The polynucleotide according to embodiment 1, wherein the at least two tandem repeats are of sequence 5'-GCCRCC-3', wherein R is any purine nucleotide.
3. The polynucleotide according to any of embodiments 1-2, wherein:
   - the 5'-UTR comprises, at its 3'-end, two tandem repeats of the sequence 5'-GCCRCC-3' operatively linked to the ORF; or alternatively,
   - the 5'-UTR comprises at its 3'-end a sequence selected from the group consisting of SEQ ID NO: 1, SEQ ID NO: 2, SEQ ID NO: 3, SEQ ID NO: 4, SEQ ID NO: 5, and SEQ ID NO: 6, operatively linked to the ORF.
4. The polynucleotide according to any of embodiments 1-3, wherein the 5'-UTR comprises or consists of, in the 5' to 3' direction, a sequence from a 5'-UTR of a transcript of a gene linked to the at least two tandem repeats of sequence 5'-GCCNCC-3'; particularly wherein the at least two tandem repeats are of sequence 5'-GCCRCC-3'.
5. The polynucleotide according to embodiment 4, wherein the sequence from a 5'-UTR of a transcript of a gene comprises a Kozak sequence, particularly a non-consensus Kozak sequence.
6. The polynucleotide according to any of embodiments 4-5, wherein the sequence from a 5'-UTR of a transcript of a gene is from a gene selected from the group consisting of apolipoprotein A2 (APOA2), hemoglobin subunit beta (HBB), and pre T cell antigen receptor alpha (PTCRA).
7. The polynucleotide according to any of embodiments 1-6, wherein the at least two tandem repeats of sequence 5'-GCCNCC-3' form a sequence selected from the group consisting of SEQ ID NO: 1, SEQ ID NO: 2, SEQ ID NO: 3, SEQ ID NO: 4, SEQ ID NO: 5, and SEQ ID NO: 6.
8. The polynucleotide according to any of embodiments 1-7, wherein the 5'-UTR comprises or consists of a sequence selected from the group consisting of SEQ ID NO: 9, SEQ ID NO: 12, and SEQ ID NO: 15.
9. A polynucleotide according to any of embodiments 1-8, further comprising one or more of:
   - a 5'-cap structure;
   - a 3' untranslated region (3'-UTR); and
   - a 3' tailing sequence.
10. The polynucleotide according to any of embodiments 1-9 comprising or consisting of in the 5' to 3' direction:
   (i) a 5'-cap structure;
   (ii) a 5' untranslated region (5'-UTR),
   (iii) an open reading frame (ORF);
   (iv) a 3' untranslated region (3'-UTR); and
   (v) a 3' tailing sequence.
11. A polynucleotide according to any of embodiments 1-10 which is an RNA polynucleotide, particularly a messenger RNA (mRNA).
12. A polynucleotide according to any one of embodiments 1-11, wherein the 5' UTR is heterologous to the ORF and/or the 3' UTR.
13. The polynucleotide according to any of embodiments 9-12, wherein the 3'-UTR comprises or consists of a sequence from a 3'-UTR of a transcript of a gene, particularly a mammalian gene.
14. The polynucleotide according to any of embodiments 9-13, wherein the 3'-UTR comprises or consists of at least two tandem repeats of a sequence from a 3'-UTR of a transcript of a gene, particularly two tandem repeats of a sequence from a 3'-UTR of a transcript of a gene.
15. The polynucleotide according to any of embodiments 9-14, wherein the 3'-UTR comprises or consists of a sequence from a 3'-UTR of a transcript of a gene selected from the group consisting of apolipoprotein A2 (APOA2), hemoglobin subunit beta (HBB), and pre T cell antigen receptor alpha (PTCRA).
16. The polynucleotide according to any of embodiments 9-15, wherein the 3'-UTR comprises or consists of a sequence selected from the group consisting of SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, and SEQ ID NO: 26.
17. A polynucleotide according to any one of embodiments 1-16, wherein the ORF encodes a polypeptide, particularly an antigen.
18. A polynucleotide according to embodiment 17, wherein the antigen is a SARS-CoV-2 antigen, particularly a SARS-CoV-2 spike antigen.
19. A polynucleotide according to any one of embodiments 9-18, wherein the 5'-cap structure is selected from the group consisting of cap-0, cap-1, cap-2, ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine; particularly the 5'-cap structure is cap-1.
20. A polynucleotide according to any one of embodiments 9-19, wherein the 3' tailing sequence is a poly-A region, particularly of sequence SEQ ID NO: 39.
21. The polynucleotide according to any of embodiments 1-20, comprising at least one of the following:
   - a 5' untranslated region (5'-UTR) of sequence SEQ ID NO: 9;
   - a 3' untranslated region (3'-UTR) of sequence SEQ ID NO: 19; and
   - a 3' tailing sequence of sequence SEQ ID NO: 39.
22. The polynucleotide according to any of embodiments 1-21 comprising or consisting of in the 5' to 3' direction:
   (i) a 5'-cap1 structure;
   (ii) a 5' untranslated region (5'-UTR) of sequence SEQ ID NO: 9;
   (iii) an open reading frame (ORF) encoding a polypeptide;
   (iv) a 3' untranslated region (3'-UTR) of sequence SEQ ID NO: 19; and
   (v) a 3' tailing sequence of sequence SEQ ID NO: 39.
23. A polynucleotide according to any one of embodiments 1-22, wherein the polynucleotide comprises at least one chemical modification, particularly wherein the chemical modification is selected from the group consisting of pseudouridine, N1-methylpseudouridine, 2-thiouridine, 4' -thiouridine, 5-methylcytosine, 2-thio- 1 -methyl- 1-deaza-pseudouridine, 2-thio- 1 -methyl -pseudouridine, 2-thio-5- aza-uridine, 2-thio-dihydropseudouridine, 2-thio-dihydrouridine, 2-thio-pseudouridine, 4-methoxy- 2-thio-pseudouridine, 4-methoxy-pseudouridine, 4-thio-i-methyl-pseudouridine, 4-thio-pseudouridine, 5-aza-uridine, dihydropseudouridine, 5-methyluridine, 5-methyluridine, 5-methoxyuridine, 2'-O-methyl uridine, N6-methyladenosine, and combinations thereof.
24. A polynucleotide according to embodiment 23, wherein the chemical modification is N1-methylpseudouridine, 5-methoxyuridine or a combination thereof; particularly wherein the chemical modification is N1-methylpseudouridine.
25. A polynucleotide according to any one of embodiments 23-24, wherein the polynucleotide is fully modified with N1-methylpseudouridine, 5-methoxyuridine or a combination thereof; particularly wherein the polynucleotide is fully modified with N1-methylpseudouridine.
26. A DNA construct comprising a promoter operatively linked to a sequence encoding a polynucleotide as defined in any of embodiments 1-25.
27. An expression vector comprising the DNA construct as defined in embodiment 26.
28. A cell comprising the polynucleotide as defined in any of embodiments 1-25, a DNA construct as defined in embodiment 26, or an expression vector as defined in embodiment 27.
29. A composition comprising a lipid nanoparticle and a polynucleotide as defined in any of embodiments 1-25, a DNA construct as defined in embodiment 26, or an expression vector as defined in embodiment 27.
30. The composition according to embodiment 29, wherein the lipid nanoparticle comprises at least one selected from the group consisting of PEG-modified lipid, a non-cationic lipid, a sterol, and an ionizable cationic lipid.
31. A pharmaceutical composition comprising a therapeutically effective amount of a polynucleotide as defined in any of embodiments 1-25, a DNA construct as defined in embodiment 26, an expression vector as defined in embodiment 27, or a composition as defined in any of embodiments 29-30, and at least one pharmaceutically acceptable excipient and/or carrier.
32. The pharmaceutical composition according to embodiment 31 which is a vaccine, optionally, further comprising an adjuvant.
33. A polynucleotide according to any of embodiments 1-25, a DNA construct according to embodiment 26, an expression vector according to embodiment 27, a cell according to embodiment 28, a composition according to any of embodiments 29-30, or the pharmaceutical composition according to any of embodiments 31-32, for use in medicine, for use in a method of inducing an immune response in a subject, for use in a method for the therapeutic immunization of a subject, or for use as a vaccine or for use in gene therapy.
34. The polynucleotide, the DNA construct, the expression vector, the composition, or the pharmaceutical composition for use according to embodiment 33, which is for use in the prevention and/or treatment of COVID-19.
35. An *in vitro* method of producing a polypeptide in a cell, the method comprising contacting the cell with a polynucleotide as defined in any of embodiments 1-25, a DNA construct as defined in embodiment 26, an expression vector as defined in embodiment 27, a composition as defined in embodiments 29-30, or a pharmaceutical composition as defined in any of embodiments 31-32.
36. A method to increase the translation rate of a polynucleotide comprising in the 5' to 3' direction a 5' untranslated region (5'-UTR) and an open reading frame (ORF), the method comprising the step of inserting at least two tandem repeats of sequence 5'-GCCNCC-3' at the 3'-end of the 5'-UTR, particularly inserting two tandem repeats of sequence 5'-GCCRCC-3' at the 3'-end of the 5'-UTR.

### Citation List

Altschul et al., "Basic local alignment search tool", 1990, J. Mol. Biol, v. 215, pages 403-410
EMBOSS: The European Molecular Biology Open Software Suite, Rice et al., 2000, Trends Genet. 16: 276-277
Hassett, K. J. et al., "Optimization of Lipid Nanoparticles for Intramuscular Administration of mRNA Vaccines", 2019, Mol. Ther. Nucleic Acid, vol. 15, pp. 1-11

## Claims

1. An artificial polynucleotide comprising in the 5' to 3' direction:
- a 5' untranslated region (5'-UTR), and
- an open reading frame (ORF),
wherein the 5'-UTR comprises, at its 3'-end, at least two tandem repeats of sequence 5'-GCCNCC-3' operatively linked to the ORF, wherein N is any nucleotide.

2. The polynucleotide according to claim 1, wherein the at least two tandem repeats are of sequence 5'-GCCRCC-3', wherein R is a purine nucleotide.

3. The polynucleotide according to any of claims 1-2, wherein the 5'-UTR comprises, at its 3'-end, two tandem repeats of sequence 5'-GCCRCC-3' operatively linked to the ORF; particularly wherein the two tandem repeats form the sequence SEQ ID NO: 3.

4. The polynucleotide according to any of claims 1-3, wherein the 5'-UTR comprises, in the 5' to 3' direction, a sequence from a 5'-UTR of a transcript of a gene linked to the at least two tandem repeats of sequence 5'-GCCNCC-3'.

5. The polynucleotide according to any of claims 1-4, wherein the sequence from a 5'-UTR of a transcript of a gene comprises a Kozak sequence at its 3'-end, particularly a non-consensus Kozak sequence.

6. The polynucleotide according to any of claims 4-5, wherein the sequence from a 5'-UTR of a transcript of a gene is from a gene selected from the group consisting of apolipoprotein A2 (APOA2), hemoglobin subunit beta (HBB), and pre T cell antigen receptor alpha (PTCRA).

7. The polynucleotide according to any of claims 1-6, wherein the 5'-UTR comprises a sequence selected from the group consisting of SEQ ID NO: 9, SEQ ID NO: 12, and SEQ ID NO: 15, or a variant thereof at least 85% identical to SEQ ID NO: 9, SEQ ID NO: 12, or SEQ ID NO: 15.

8. A polynucleotide according to any of claims 1-7, further comprising one or more of:
- a 5'-cap structure;
- a 3' untranslated region (3'-UTR); and
- a 3' tailing sequence.

9. The polynucleotide according to any of claims 1-8 comprising in the 5' to 3' direction:
(i) a 5'-cap structure, particularly a cap-1 structure;
(ii) a 5' untranslated region (5'-UTR), particularly of sequence SEQ ID NO: 9;
(iii) an open reading frame (ORF), particularly, encoding a polypeptide;
(iv) a 3' untranslated region (3'-UTR), particularly of sequence SEQ ID NO: 19; and
(v) a 3' tailing sequence, particularly of sequence SEQ ID NO: 39.

10. A polynucleotide according to any of claims 1-9 which is an RNA polynucleotide, particularly a messenger RNA (mRNA).

11. A polynucleotide according to any one of claims 1-10, wherein the polynucleotide comprises at least one chemical modification, particularly wherein the chemical modification is selected from the group consisting of pseudouridine, N1-methylpseudouridine, N6-methyladenosine, 2-thiouridine, 4'-thiouridine, 5-methylcytosine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-I-methyl-pseudouridine, 4-thio-pseudouridine, 5-aza-uridine, dihydropseudouridine, 5-methyluridine, 5-methyluridine, 5-methoxyuridine, 2'-O-methyluridine, and combinations thereof.

12. A DNA construct comprising a promoter operatively linked to a sequence encoding a polynucleotide as defined in any of claims 1-11.

13. A composition comprising:
- a lipid nanoparticle; and
- a polynucleotide as defined in any of claims 1-11 or a DNA construct as defined in claim 12.

14. A pharmaceutical composition comprising a therapeutically effective amount of the polynucleotide according to any of claims 1-11, the DNA construct according to claim 12, or the composition according to claim 13, and at least one pharmaceutically acceptable excipient and/or carrier.

15. An *in vitro* method of producing a polypeptide in a cell, the method comprising contacting the cell with a polynucleotide as defined in any of claims 1-11, a DNA construct as defined in claim 12, a composition as defined in claim 13, or a pharmaceutical composition as defined in claim 14.

16. A method to increase the translation rate of a polynucleotide comprising, in the 5' to 3' direction, a 5' untranslated region (5'-UTR) and an open reading frame (ORF), the method comprising the step of inserting at least two tandem repeats of sequence 5'-GCCNCC-3' at the 3'-end of the 5'-UTR, wherein N is any nucleotide.
